**Europäisches Patentamt**

**European Patent Office**

**Office européen des brevets**

(19)

(11) Veröffentlichungsnummer: **0 171 024 B1**

## (12) EUROPÄISCHE PATENTSCHRIFT

(45) Veröffentlichungstag der Patentschrift: **11.09.91**

(21) Anmeldenummer: **85109567.9**

(22) Anmeldetag: **30.07.85**

(51) Int. Cl.⁵: **C12N 15/62**, C12N 15/72, C12P 21/02, C12P 19/34, C07K 7/10, C12N 1/20, //C12R1/19

(54) Gentechnologisches Verfahren zur Herstellung von Hirudinen und Mittel zur Durchführung dieses Verfahrens.

(30) Priorität: **10.08.84 DE 3429430**

(43) Veröffentlichungstag der Anmeldung:
**12.02.86 Patentblatt 86/07**

(45) Bekanntmachung des Hinweises auf die Patenterteilung:
**11.09.91 Patentblatt 91/37**

(84) Benannte Vertragsstaaten:
**AT BE CH DE FR GB IT LI LU NL SE**

(56) Entgegenhaltungen:
**EP-A- 0 142 860**
**EP-A- 0 158 564**
**EP-A- 0 168 342**

**FEBS LETTERS, vol. 165, Nr. 2, 9. Janner 1984, Amsterdam, New York J. DOOT et al. "The complete amino acid sequence of hirudin, a thrombin specific inhibitor" Seiten 180-184**

(73) Patentinhaber: **HOECHST AKTIENGESELL-SCHAFT**
**Postfach 80 03 20**
**W-6230 Frankfurt am Main 80(DE)**

(72) Erfinder: **Brauer, Dieter, Dr.**
**Berliner Strasse 14**
**W-6093 Flörsheim am Main(DE)**
Erfinder: **Engels, Joachim, Prof. Dr.**
**Feldbergstrasse 1**
**W-6242 Kronberg/Taunus(DE)**
Erfinder: **Habermann, Paul, Dr.**
**Heimchenweg 80**
**W-6230 Frankfurt am Main 80(DE)**
Erfinder: **Uhlmann, Eugen, Dr.**
**Washington Street 287**
**Belmont, Mass. 02178(US)**
Erfinder: **Wengenmayer, Friedrich, Dr.**
**Am Seyenbach 38**
**W-6238 Hofheim am Taunus(DE)**

**Beschreibung**

Hirudin ist ein aus Hirudo medicinalis gewonnenes Polypeptid, das eine spezifische Antithrombin-Aktivität zeigt und als Antikoagulans dient.

Es wurde nun gefunden, daß sich Polypeptide der allgemeinen Formel I

$$(X)_m-A-B-C-Tyr-D-Asp-Cys-E-Glu-Ser-Gly-Gln-Asn-Leu-Cys-Leu-$$
$$-Cys-Glu-Gly-Ser-Asn-Val-Cys-Gly-Gln-Gly-Asn-Lys-Cys-Ile-$$
$$-Leu-Gly-Ser-Asp-F-G-Lys-Asn-Gln-Cys-Val-Thr-Gly-Glu-$$
$$-Gly-Thr-Pro-Lys-Pro-Gln-Ser-His-Asn-Asp-Gly-Asp-Phe-Glu-$$
$$-H-Ile-Pro-Glu-Glu-Tyr-Leu-Gln-(Z)_n-OH$$

$$(I)$$

in welcher

m = 0 - 50,
n = 0 - 100 und
X für gleiche oder verschiedene Reste genetisch codierbarer Aminosäuren steht,
A Ile oder eine direkte Bindung,
B Ile, Thr oder eine direkte Bindung,
C Thr, Val, Ile, Leu oder Phe,
D Thr oder eine direkte Bindung,
E Thr oder Ile,
F Gly oder eine direkte Bindung,
G Glu oder eine direkte Bindung,
H Glu oder Pro bedeuten, und
Z für gleiche oder verschiedene Reste genetisch codierbarer Aminosäuren steht und

in der gegebenenfalls jeweils 2 der 6 Cys-Reste über Disulfid-Brücken verknüpft sind, auch gentechnologisch herstellen lassen, wenn man in ein Expressionsplasmid ein Gen einbaut, das für ein Polypeptid der Formel I oder Teilsequenzen davon codiert. Dieses wird im folgenden auch als "Hirudin" bezeichnet.

Das erforderliche Gen kann nach bekannten Methoden chemisch synthetisiert, aus dem Genom isoliert und prozessiert werden oder aus induzierten Zellen nach bekannten Methoden die mRNA isoliert und hieraus die cDNA gewonnen werden.

Bevorzugt ist der Weg über die cDNA und vor allem die chemische Synthese, insbesondere nach der Phosphit-Methode. Bevorzugt ist weiterhin die Synthese eines Gens, welches für das Polypeptid der Formel I codiert, in welcher m 1 und n Null ist, X für Met oder Arg, B für Thr oder eine direkte Bindung, C für Thr und G für Glu stehen. Eine weitere bevorzugte Ausführungsform der Erfindung betrifft Polypeptide der Formel I, in der $(X)_m$-A-B-C- nicht für Val-Val steht, wenn n Null ist, D und E Thr, F Gly und G und H Glu bedeuten.

Besonders bevorzugt ist die Synthese von Polypeptiden der Formel I, in denen m 1 ist, X Met oder Arg, A und B direkte Bindungen, C, D und E Thr, F Gly, G und H Glu und n Null bedeuten. Die hierfür bevorzugte DNA-Sequenz I ist im Anhang wiedergegeben und dient im folgenden zur Erläuterung der Erfindung.

Der genetische Code ist bekanntlich "entartet", d.h. daß nur für zwei Aminosäuren eine einzige Nucleotid-Sequenz codiert, während den restlichen 18 genetisch codierbaren Aminosäuren 2 bis 6 Tripletts zuzuordnen sind. Von den hierdurch gegebenen Variationsmöglichkeiten machen außerdem die Wirtszellen unterschiedlicher Spezies nicht immer den gleichen Gebrauch. Für die Synthese der Gene besteht somit eine unübersehbare Vielfalt von Codon-Möglichkeiten.

Es wurde nun gefunden, daß die DNA-Sequenz I (Anhang), die für die gesamte Aminosäurensequenz codiert, sowie die zur Synthese der Sequenz I benutzten DNA-Teilsequenzen II A und II B besonders vorteilhaft für die gentechnologische Synthese der besonders bevorzugten Form des Hirudin sind. Am 5'-Ende des codierenden Stranges der DNA-Sequenz I befindet sich eine "überhängende" DNA-Sequenz, entsprechend der Restriktionsendonuclease XbaI, am 3'-Ende des codierenden Stranges dagegen die einzelsträngige, überhängende Sequenz entsprechend dem Restriktionsenzym Sal I. Diese beiden unterschiedlichen Erkennungssequenzen gewährleisten die Insertion der DNA in Plasmide in der gewünschten Orientierung. (Es ist aber auch möglich, gleiche Erkennungssequenzen zu wählen und nach Charakterisierung der Plasmid-DNA mittels entsprechender Restriktions- oder DNA-Sequenzanalyse oder durch Expression eine entsprechende Selektion vorzunehmen).

Zwischen diesen Erkennungssequenzen und den Codons für die Aminosäurefolge befindet sich am 5'-Ende des codierenden Stranges das Codon für die Aminosäure Methionin (das in der DNA-Sequenz I mit 0 beziffert ist). Alternativ hierzu kann eine Praesequenz (auch Signal- oder leader-Sequenz genannt) eines bakteriellen oder sonstigen wirtseigenen Proteins stehen (Übersichtsartikel: Perlman und Halvorson; J. Mol. Biol. 167 (1983), 391), welche die Sekretion des gewünschten Polypeptids aus dem Cytoplasma bewirkt und bei diesem Exkretionsprozeß von einer in der Wirtszelle natürlich vorkommenden Signal-Peptidase abgespalten wird. Am Ende des codierenden Stranges folgen dann erfindungsgemäß auf das für Glutamin codierende Triplett ein Stop-Codon oder bevorzugt - wie in der DNA-Sequenz I dargestellt - zwei Stop-Tripletts. Zwischen diesen stop-codons und dem überhängenden SalI-Ende ist zusätzlich in der DNA-Sequenz I eine Nucleotidsequenz entsprechend dem Restriktionsenzym SstI eingebaut.

Eine interne singuläre Schnittstelle für das Restriktionsenzym Bam HI (im Codon 30/31) ermöglicht die Subklonierung zweier Genfragmente HIR-I und HIR-II (siehe DNA-Sequenz II), die in gut untersuchte Klonierungsvektoren, wie etwa pBR 322, pUC 8 oder pUC 12, eingebaut werden können. Zusätzlich wurden innerhalb des Gens eine Reihe von weiteren singulären Erkennungssequenzen für Restriktionsenzyme eingebaut, die einerseits einen Zugang zu Teilsequenzen des Hirudin schaffen und andererseits die Durchführung von Variationen erlauben (Tabelle 1):

### Tabelle 1:

| Restriktions-enzym | Schnitt nach Nucleotid-Nr. (codierender Strang) |
|---|---|
| Rsa I[a] | 8 |
| Acc I | 12 |
| Hinf I | 27 |
| Xho II[a] | 57 |
| Fnu 4 HI | 71 |
| Hae III | 73 |
| Bst NI | 75 |
| Hph I | 117 |
| Rsa I[b] | 137 |
| Kpn I | 139 |
| Taq I | 172 |
| Xho II[b] | 178 |
| Dde I | 184 |
| Mbo II | 186 |
| Sst I | 210 |

a) singulär bezüglich der Teilsequenz HIR-I
b)    "          "          "          "          HIR-II

Die DNA-Sequenz I läßt sich aus 14 Oligonucleotiden mit einer Länge von 25 bis 35 Nucleotiden aufbauen (siehe DNA-Sequenz II),indem diese zunächst chemisch synthetisiert und dann über "sticky ends" von 4 bis 6 Nucleotiden enzymatisch verknüpft werden.

Bei der DNA-Sequenz I wurde weiterhin berücksichtigt, daß bei denjenigen Aminosäuren, denen mehrere Codons zuzuordnen sind, diese nicht gleichwertig sind, sondern vielmehr in der jeweiligen Wirtszelle wie E. coli unterschiedliche Präferenzen zeigen. Weiterhin wurden palindromische Sequenzen auf ein Mindestmaß reduziert.

Die Genstruktur der DNA-Sequenz I ist somit leicht aus relativ kleinen Bausteinen zugänglich, ermöglicht die Subklonierung zweier Genfragmente in gut bekannte Vektoren und erlaubt deren Kombination zum Gesamtgen sowie gegebenenfalls Veränderungen derselben.

Je nach Einbau des synthetischen Gens in den Klonierungsvektor wird das erwünschte Peptid mit der Aminosäuresequenz des Hirudins unmittelbar (DNA-Sequenz IC) oder in Form eines Fusionsproteins mit einem bakteriellen Protein wie β-Galactosidase oder Partialsequenzen desselben exprimiert. Solche Fusionsproteine können dann in bekannter Weise chemisch oder enzymatisch gespalten werden. Steht beispielsweise in der Position 0 der Sequenz I die Aminosäure Methionin (DNA-Sequenz IA), so kann eine chemische Spaltung mit Bromcyan erfolgen, steht in dieser Position die Aminosäure Arginin (DNA-Sequenz IB), so kann eine enzymatische Spaltung mit Trypsin erfolgen.

Bevorzugte Variationen der Aminosäuresequenzen sind in der Tabelle 2 dargestellt, wobei m und n Null sind:

## Tabelle 2:

|     | A   | B   | C   | D   | E   | F   | G   | H   |
| --- | --- | --- | --- | --- | --- | --- | --- | --- |
| a)  | Ile | Thr | Thr | Thr | Thr | Gly | Glu | Glu |
| b)  | Ile | –   | Thr | Thr | Thr | Gly | Glu | Glu |
| c)  | Ile | –   | Thr | Thr | Ile | Gly | Glu | Glu |
| d)  | Ile | –   | Thr | Thr | Thr | Gly | Glu | Pro |
| e)  | Ile | –   | Thr | Thr | Ile | Gly | Glu | Pro |
| f)  | –   | –   | Thr | –   | Thr | Gly | –   | Glu |
| g)  | –   | –   | Thr | Thr | Thr | Gly | –   | Glu |

Diese Modifikationen lassen sich nach Zusammensetzung des synthetischen Gens auf DNA-Ebene leicht durch Austausch der entsprechenden Genfragmente gegen de-novo-synthetisierte DNA-Sequenzen unter Ausnutzung entsprechender Restriktionsenzym-Schnittstellen realisieren.

Als Beispiel für eine Variation der Aminosäuresequenz sei das (bekannte) Polypeptid der Formel I genannt, in der m 1 ist, X und C für Val stehen, A und B direkte Bindungen darstellen, D und E Thr, F Gly, G und H Glu und n Null bedeuten. Diese Modifikation läßt sich auf der DNA-Ebene leicht über die Schnittstelle entsprechend dem Restriktionsenzym Acc I realisieren.

Der Einbau der synthetischen Gene bzw. Genfragmente in Klonierungsvektoren, beispielsweise in die handelsüblichen Plasmide pUC 8, pUC 12 und pBR 322 bzw. andere allgemein zugängliche Plasmide wie ptac 11 und pKK 177.3, erfolgt in an sich bekannter Weise. Auch können die chemisch synthetisierten Gene zuvor mit geeigneten chemisch synthetisierten Kontrollregionen versehen werden, die eine Expression der Proteine ermöglichen. Hierzu kann auf das Lehrbuch von Maniatis (Molecular Cloning, Maniatis et al., Cold Spring Harbor, 1982) verwiesen werden. Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben. Die Gewinnung des exprimierten Proteins und dessen Reinigung können nach an sich bekannten Verfahren erfolgen.

Die erfindungsgemäß erhaltenen Genfragmente HIR-I und HIR-II, die damit erhaltenen Hybridplasmide und die transformierten Wirtsorganismen sind neu und Gegenstand der Erfindung. Dasselbe gilt für aus der DNA-Sequenz I abgewandelte neue DNA-Sequenzen. Weitere Ausgestaltungen der Erfindung sind in den Patentansprüchen niedergelegt.

In den folgenden Beispielen werden noch einige Ausgestaltungen der Erfindung im einzelnen erläutert, woraus sich die Vielzahl der möglichen Abwandlungen und Kombinationen für den Fachmann ergeben. Prozentangaben beziehen sich hierbei auf das Gewicht, wenn nichts anderes angegeben ist.

Beispiele

1. Chemische Synthese eines einzelsträngigen Oligonucleotids

Am Beispiel des Genbausteins Ia, der die Nucleotide 1-32 des codierenden Strangs umfaßt, wird die Synthese der Genbausteine erläutert. Nach bekannten Methoden (M.J. Gait et al., Nucleic Acids Res. 8 (1980) 1081-1096)) wird das am 3'-Ende stehende Nucleosid, im vorliegenden Falle also Thymidin (Nucleotid Nr. 32), an Kieselgel ($^{(R)}$FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Thymidin wird als 3'-O-Succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylaminogruppe acyliert.

In den folgenden Syntheseschritten wird die Basenkomponente als 5'-O-Dimethoxytrityl-nucleosid-3'-phosphorigsäuremonomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und dar keine Aminogruppe enthaltende Thymin ohne Schutzgruppe vorliegen.

50 mg des polymeren Trägers, der 2 $\mu$mol Thymidin gebunden enthält, werden nacheinander mit den folgenden Agentien behandelt:

a) Nitromethan,
b) gesättigte Zinkbromidlösung in Nitromethan mit 1 % Wasser,
c) Methanol,
d) Tetrahydrofuran,
e) Acetonitril,
f) 40 $\mu$mol des entsprechenden Nucleosidphosphits und 200 $\mu$mol Tetrazol in 0,5 ml wasserfreiem Acetonitril (5 Minuten),
g) 20 % Acetanhydrid in Tetrahydrofuran mit 40 % Lutidin und 10 % Dimethylaminopyridin (2 Minuten),
h) Tetrahydrofuran,
i) Tetrahydrofuran mit 20 % Wasser und 40 % Lutidin,
j) 3 % Jod in Kollidin/Wasser/Tetrahydrofuran im Volumenverhältnis 5:4:1,
k) Tetrahydrofuran und
l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten.

Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib-IIh synthetisiert, deren Nucleotidfolge aus der DNA-Sequenz II hervorgeht.

2. Enzymatische Verknüpfung der einzelsträngigen Oligonucleotide zu den Genfragmenten HIR-I und HIR-II

Zur Phosphorylierung der Oligonucleotide am 5'-Terminus wurde je 1 nmol der Oligonucleotide Ib bis Ie mit 5 nmol Adenosintriphosphat mit vier Einheiten T4-Polynucleotid-Kinase in 20 $\mu$l 50 mM Tris-HCl-Puffer (pH 7,6), 10 mM Magnesiumchlorid und 10 mM Dithiothreitol (DTT) 30 Minuten bei 37° C behandelt. Das Enzym wird durch fünfminütiges Erhitzen auf 95° C desaktiviert. Die Oligonucleotide Ia, If, IIa und IIh, welche in der DNA-Sequenz IIA und IIB die "überhängende" Sequenz bilden, werden nicht phosphoryliert. Dies verhindert bei der nachfolgenden Ligation die Ausbildung größerer Subfragmente als sie der DNA-Sequenz IIA oder IIB entsprechen.

Die Oligonucleotide Ia-If werden wie folgt zum Subfragment HIR-I ligiert: Je 1 mmol der Oligonucleotide Ia und If sowie der 5'-Phosphate von Ib, Ic, Id und Ie werden zusammen in 45 $\mu$l Puffer, enthaltend 50 mM Tris-HCl (pH 7,6), 20 mM Magnesiumchlorid, 25 mM Kaliumchlorid und 10 mM DTT, gelöst. Für das "Annealing" der Oligonucleotide gemäß DNA-Sequenz IIA wird die Lösung der Oligonucleotide 2 Minuten auf 95° C erhitzt und dann langsam (2-3 Stunden) auf 20° C abgekühlt. Zur enzymatischen Verknüpfung setzt man dann 2 μl 0,1 M DTT, 8 μl 2,5 mM Adenosintriphosphat (pH 7) sowie 5 μl T4-DNA-Ligase (2 000 Units) zu und inkubiert 16 Stunden bei 22° C.

Analog werden die Oligonucleotide IIb bis IIg phosphoryliert und dann mit den Oligonucleotiden IIa und IIh zusammen zum Subfragment HIR-II ligiert.

Die Reinigung der Genfragmente HIR-I und HIR-II erfolgt durch Gelelektrophorese auf einem 10%igen Polyacrylamidgel (ohne Harnstoffzusatz, 20 · 40 cm, 1 mm Dicke), wobei als Markersubstanz ØX 174 DNA (Fa. BRL), geschnitten mit Hinf I, oder pBR 322, geschnitten mit Hae III, dient.

3. Herstellung von Hybridplasmiden, die die Genfragmente HIR-I und HIR-II enthalten

a) Einbau des Genfragmentes HIR-I in pUC 12

Das handelsübliche Plasmid pUC 12 wird in bekannter Weise mit den Restriktionsendonucleasen Xba I und Bam HI nach den Angaben der Hersteller geöffnet. Der Verdauungsansatz wird auf einem 5%igen Polyacrylamidgel durch Elektrophorese in bekannter Weise aufgetrennt und die Bruchstücke durch Anfärben mit Ethidiumbromid oder durch radioaktive Markierung ("Nick-Translation" nach Maniatis, a.a.O.) sichtbar gemacht. Die Plasmidbande wird anschließend aus dem Acrylamidgel ausgeschnitten und elektrophoretisch vom Polyacrylamid abgetrennt. Die Auftrennung des Verdauungsansatzes kann auch auf 2%igen niedrigschmelzenden Agarosegelen (wie in Beispiel 5a) beschrieben) erfolgen.

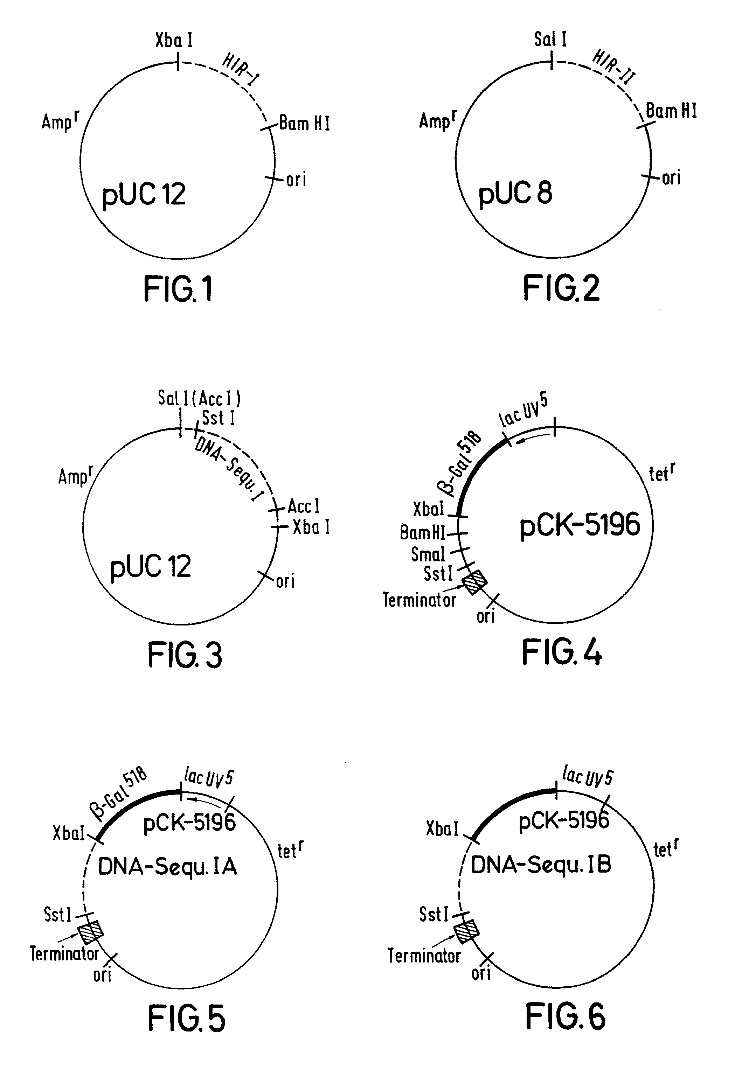

1 μg Plasmid wird dann mit 10 ng Genfragment HIR-I, das zuvor wie in Beispiel 2 beschrieben phosphoryliert wurde, über Nacht bei 16° C ligiert. Man erhält das Hybridplasmid gemäß Figur 1.

b) Einbau des Genfragments HIR-II in pUC 8

Analog zu a) wird das handelsübliche Plasmid pUC 8 mit Bam HI und Sal I aufgeschnitten und mit dem Genfragment HIR-II, das zuvor wie in Beispiel 2 beschrieben phosphoryliert wurde, ligiert. Man erhält das Hybridplasmid gemäß Figur 2.

4. Synthese des kompletten Gens und Einbau in ein Plasmid

a) Transformation und Amplifikation

Die so erhaltenen Hybridplasmide werden in E. coli transformiert. Hierzu wird der Stamm E. coli K 12 durch Behandlung mit einer 70 mM Calciumchloridlösung kompetent gemacht und mit der Suspension des Hybridplasmids in 10 mM Tris-HCl-Puffer (pH 7,5), der 70 mM an Calciumchlorid ist, versetzt. Die transformierten Stämme werden wie üblich unter Ausnutzung der durch das Plasmid vermittelten Antibiotikaresistenzen bzw. -empfindlichkeiten selektioniert und die Hybridvektoren amplifiziert. Nach Abtöten der Zellen werden die Hybridplasmide isoliert, mit den ursprünglich eingesetzten Restriktionsenzymen aufgeschnitten und die Genfragmente HIR-I und HIR-II durch Gelelektrophorese isoliert.

b) Verknüpfung der Genfragmente

Die durch Amplifikation erhaltenen Subfragmente HIR-I und HIR-II werden wie im Beispiel 2 beschrieben (Annealing bei 60° C) enzymatisch verknüpft und das so erhaltene synthetische Gen mit der DNA-Sequenz I in den Klonierungsvektor pUC 12 eingeführt. Man erhält ein Hybridplasmid gemäß Figur 3.

c) Herstellung eines Gens für Val[1], Val[2] -Hirudin

Aus dem Plasmid gemäß Figur 3 wird wie in Beispiel 5a) beschrieben das AccI-SalI-Fragment (Nucleotide 13-212) erhalten, welches dann mit Hilfe des folgenden Adaptors

```
5'  CT AGA ATG GTT GTA T        3'
3'         T TAC CAA CAT ATA     5'
        XbaI                AccI
```

in das Plasmid pUC 12, das zuvor mit XbaI und SalI geöffnet wurde, ligiert werden kann.

d) Herstellung eines Gens für die Variante a) der Tabelle 2

Hierzu wird die folgende DNA-Sequenz synthetisiert:

```
            Glu   Phe   Met   Ile   Thr   Thr
   5' GG    GAA   TTC   ATG   ATC   ACA   ACG   T        3'
      CC    CTT   AAG   TAC   TAG   TGT   TGC   ATA
      SmaI                                     AccI
```

Diese Sequenz kann als SmaI-AccI-Adaptor in die mit den Restriktionsenzymen Smal und Accl geöffnete DNA aus dem Plamid gemäß Figur 3 eingebaut werden.

e) Herstellung eines Gens für die Variante b) der Tabelle 2

Zunächst wird die folgende DNA-Sequenz synthetisiert:

```
            Glu   Phe   Met   Ile   Thr
   5'  GG   GAA   TTC   ATG   ATC   ACG   T     3'
       CC   CTT   AAG   TAC   TAG   TGC   ATA
       SmaI                              AccI
```

Diese um das ACA-Triplett verkürzte DNA-Sequenz wird wie unter d) beschrieben eingebaut.

f) Herstellung eines Gens für die Variante c) der Tabelle 2

Man geht von der DNA-Sequenz aus, wie sie vorstehend unter e) beschrieben ist (Variante b) der Tabelle 2). Nach Spaltung mit Accl und Sall erhält man zwei Fragmente, die isoliert werden. Das Accl-Sall-(Hirudin)-Fragment wird mit dem Enzym Hinfl geschnitten und das größere der erhaltenen Fragmente isoliert. Dieses Fragment, die mit AccI-Sall-geöffnete Restplasmid-DNA und die synthetische DNA-Sequenz

```
            Thr   Asp   Cys   Ile
   5'  AT   ACT   GAC   TGC   ATC   G       3'
       TGA  CTG   ACG   TAG   CTT   A
       AccI                    HinfI
```

werden im gleichen Gefäß ligiert. Das erhaltene Hybridplasmid kodiert für die Variante c) der Tabelle 2.

g) Herstellung eines Gens für die Variante d) der Tabelle 2

Man geht ebenfalls von der DNA-Sequenz aus, wie sie vorstehend unter e) beschrieben ist (also für die Variante b) der Tabelle 2 kodiert), die mit Accl und Sall geschnitten wird. Das Accl-Sall-(Hirudin)-Fragment wird aber mit Taql und Ddel umgesetzt und das herausgespaltene Fragment durch die synthetische DNA-Sequenz

```
            Glu   Pro   Ile
   5'  C    GAA   CCG   ATC   CC     3'
            TT    GGC   TAG   GGA   CT
       TaqI                    Dde I
```

ersetzt. Das derart veränderte Accl-Sall-(Hirudin)-Fragment wird nun mit der Restplasmid-DNA ligiert. Das erhaltene Plasmid kodiert für die Variante d) der Tabelle 2.

h) Herstellung eines Gens für die Variante g) der Tabelle 2

Aus dem Hybridplasmid gemäß Figur 2 wird das BamHI-Sall-(Hirudin)-Fragment isoliert und mit Kpnl nachgeschnitten. Das größere der beiden entstandenen Fragmente wird isoliert und mit der synthetischen DNA-Sequenz

```
        Ser   Asp   Gly   Lys   Asn   Gln   Cys   Val
  5'  GA  TCC   GAC   GAA   AAG   AAC   CAG   TGC   GTT
         G    CTG   CTT   TTC   TTG   GTC   ACG   CAA

  BamHI
```

```
        Thr   Gly   Glu   Gly
  5'  ACT   GGC   GAA   GGT   AC        3'
      TGA   CCG   CTT   C       Kpn I
```

ligiert. Das Ligationsprodukt wird nun mit dem Xbal-BamHI-(Hirudin)-Fragment, isoliert aus Plasmid-DNA gemäß Figur 1, verknüpft und - wie unter 4b) beschrieben - in pUC 12 eingeführt. Man erhält ein Hybridplasmid, das für die Variante g) der Tabelle 2 kodiert.

i) Herstellung eines Gens für die Variante f) der Tabelle 2

Aus der DNA, die für die Variante g) der Tabelle 2 kodiert, wird das Xbal-Sall-(Hirudin)-Fragment isoliert und mit Hinfl umgesetzt. Das größere der beiden entstandenen Fragmente wird dann mit der synthetischen DNA-Sequenz

```
         Arg   Met   Thr   Tyr   Asp   Cys   Thr
  5'  CT  AGA   ATG   ACG   TAT   GAC   TGC   ACT   G      3'
         T    TAC   TGC   ATA   CTG   ACG   TGA   CTT   A
  XbaI                                       Hinf I
```

ligiert und das Ligationsprodukt in das mit Xbal und Sall geöffnete Plasmid pUC 12 inseriert. Das Hybridplasmid kodiert für die Variante f) der Tabelle 2.

j) Herstellung weiterer Varianten

Als Beispiele für weitere mögliche Varianten seien die folgenden angeführt:

Die DNA-Sequenzen, die für die Varianten c) und d) der Tabelle 2 kodieren, enthalten die BamHI-Erkennungsstelle entsprechend Position 97 bp der DNA-Sequenz I. Unter Ausnutzung dieser Schnittstelle lassen sich nun die vorstehend beschriebenen Accl-BamHI- bzw. BamHI-Sall-Teilsequenzen der beiden Varianten beliebig miteinander verknüpfen.

Alle diese Varianten können, analog wie für die DNA-Sequenz I beschrieben, in E. coli transformiert und exprimiert werden.

5. Konstruktion von Hybridplasmiden für die Expression der DNA-Sequenzen IA, IB und IC

a) Einbau der DNA-Sequenz IC in pKK 177.3 (Direktexpression)

Das Expressionsplasmid pKK 177.3 (Plasmid ptac 11, Amman et al., Gene 25 (1983) 167, bei dem in die Eco RI-Erkennungsstelle synthetisch eine Sequenz eingebaut wurde, die eine Sal I-Schnittstelle enthält) wird mit den Restriktionsenzymen Eco RI und Sal I geöffnet. Aus dem Plasmid gemäß Figur 3 wird die DNA-Sequenz IC folgendermaßen gewonnen. Man schneidet das Plasmid zuerst mit dem Restriktionsenzym Sal I, dann mit dem Enzym Acc I und trennt dann das kleine Acc I - Sal I-Fragment durch Gelelektrophorese auf einem 2%igen niedrig schmelzenden Agarosegel von der Plasmidbande ab, indem man die DNA durch Auflösen des Gels bei erhöhter Temperatur (nach Maßgabe der Hersteller) wiedergewinnt. Dieses DNA-Fragment läßt sich mit Hilfe des folgenden Adaptors

```
5' AATTC ATG ACG T    3'
3'      G TAC TGC ATA 5'
Eco RI                Acc I
```

zur DNA-Sequenz IC umsetzen.

Durch Ligation des aufgeschnittenen Plasmids pKK 177.3 mit dem Gen IC wird ein Hybridplasmid geschaffen, bei dem der Insertion eine Expressions- bzw. Regulationsregion vorgeschaltet ist. Nach Zugabe eines geeigneten Induktors wie Isopropyl-$\beta$-thiogalactopyranosid (IPTG) wird eine mRNA gebildet, die zur Expression des Methionyl-Polypeptids entsprechend der DNA-Sequenz IC führt.

b) Einbau der Hirudin-Gene IA bzw. IB in das Expressionsplasmid pCK-5196 (Fusionskonstruktion)

Das Expressionsplasmid pCK-5196 (Fig. 4) ist ein Derivat des Plasmids pAT 153 (Twigg u. Sherrat), welches selbst ein "high-copy-number"-Derivat des bekannten Plasmids pBR 322 darstellt. Es enthält den bekannten Lac UV$^5$-Promotor mit der bekannten Sequenz der $\beta$-Galaktosidase bis Nukleodid Nr. 1554 (entsprechend Aminosäure Nr. 518:Trp), eingesetzt zwischen das tet$^R$-Gen (Hind III von pBR 322) und den Terminator des amp$^R$-Gens (Aha III in Pos. 3231 in pBR 322). Ein weiteres Merkmal dieses Vektors ist die zwischen die $\beta$-Galaktosidasesequenz und die Terminatorsequenz eingesetzte, aus dem bekannten Plasmid pUC 13 stammende Polylinker-Sequenz. Die Transkription des lac UV$^5$-Promoters läuft gegen das tet$^R$-Gen ab.

Dieses Plasmid enthält bei der Aminosäure Nr. 518 der $\beta$-Galactosidase den Polylinkeranteil Xba I-Bam HI-Sma I-Sst I aus dem Plasmid pUC 13, der als Klonierungsstelle für eukaryotische Gene dient. Das Plasmid pCK-5196 wird mit Hilfe der Restriktionsenzyme Xba I und Sst I geöffnet und mit der DNA-Sequenz IA ligiert, welche man aus dem Plasmid gemäß Fig. 3 durch Xba I-Sst I-Verdauung isolieren kann. Man erhält ein Hybridplasmid gemäß Fig. 5, das hinter der lac UV$^5$-Kontrollregion die Codons für die ersten 518 Aminosäuren der $\beta$-Galactosidase und daran anschließend die Codons für Ser-Arg-Met-(Hirudin 1-64) enthält.

Auf gleiche Weise kann man ein Hybridplasmid gem. Fig. 6 erhalten, welches im Plasmid pCK-5196 die Insertion mit der DNA-Sequenz IB enthält, bei welcher dem Codon für die Aminosäure Nr. 1 (Threonin) nun aber das Codon für die Aminosäure Arginin vorangestellt ist. Die DNA-Sequenz IB erhält man aus dem Plasmid gemäß Fig. 3, indem man dieses mit den Restriktionsenzymen Sst 1 und Acc I schneidet und mit dem folgenden Adaptor ligiert:

```
5' CT AGA CGT ACG   T    3'
3'        T GCA TGC   ATA 5'
   Xba I              Acc I
```

6. Transformation der Hybridplasmide.

Kompetente E. coli-Zellen werden mit 0,1 bis 1 $\mu$g der Hybridplasmide, die die Sequenz I bzw. IA, IB oder IC enthalten, transformiert und im Falle der tac-Plasmide auf Ampicillin enthaltenden Agarplatten, im Falle der pCK-5196 Hybridplasmide auf Tetracyclin enthaltenden Agarplatten plattiert. Anschließend lassen sich Klone, die die korrekt integrierten Hirudin-Sequenzen in den entsprechenden Plasmiden enthalten, durch DNA-Schnellaufarbeitung identifizieren (Maniatis, a.a.O.).

7. Expression der Hirudin-Aktivität aufweisenden Polypeptide

Nach Transformation der genannten tac-Hybridplasmide in E. coli wird ein Polypeptid exprimiert, das außer der Hirudin-Aminosäuresequenz am Aminoterminus noch eine zusätzliche Methionylgruppe trägt, welche aber durch Bromcyanspaltung eliminierbar ist. Dagegen erhält man nach der Transformation von E. coli mit dem Hybridplasnid gemäß Fig. 5 bzw. Fig. 6 Fusionsproteine aus 518 Aminosäuren der $\beta$-Galactosidase und Hirudin, die über die Sequenz Ser-Arg-Met bzw. Ser-Arg-Arg miteinander verbrückt sind. Diese Fusionsproteine lassen sich mit Bromcyan bzw. mit Trypsin zu Hirudin und $\beta$-Galactosidase-Fragmenten spalten.

8. Aufarbeitung und Reinigung

Die zur gewünschten optischen Dichte kultivierten Bakterienstämme werden mit einem geeigneten Induktor, beispielsweise IPTG, hinreichend lange, beispielsweise 2 Stunden, inkubiert. Anschließend werden die Zellen mit 0,1 % Kresol und 0,1 mM Benzylsulfonylfluorid abgetötet.

Nach Zentrifugieren oder Filtrieren wird die Zellmasse in einer Pufferlösung (50 mM Tris, 50 mM EDTA, pH 7,5) aufgenommen und mechanisch aufgeschlossen, beispielsweise mit einer French-Presse bzw. (R)DYNO-Mühle (Fa. Willy Bachofer, Basel), worauf die unlöslichen Bestandteile abzentrifugiert werden. Aus dem Überstand wird das das Hirudin enthaltende Protein nach üblichen Verfahren gereinigt. Geeignet sind Ionenaustauscher-, Adsorptions-, Gelfiltrationssäulen oder Affinitätschromatographie an Thrombin- oder Antikörpersäulen. Durch Natriumdodecylsulfat-Acrylamidgel-oder HPLC-Analytik werden Anreicherung und Reinheit des Produktes kontrolliert. Fusionsproteine mit einem $\beta$-Galactosidase-Anteil lassen sich bereits im Rohextract der lysierten Bakterien anhand ihres unterschiedlichen Laufverhaltens von der $\beta$-Galactosidase (1-518) nachweisen. Die Charakterisierung des gentechnologisch erzeugten Hirudins erfolgt durch Vergleich mit der aus Blutegeln isolierten, authentischen Substanz bzw. mit Hilfe von Tests, die auf den blutgerinnungshemmenden Eigenschaften des Hirudins beruhen.

DNA-Sequenz I

```
Triplett Nr.                        0    1    2    3    4    5
Aminosäure                         Met  Thr  Tyr  Thr  Asp  Cys
Nucleotid Nr.          1            10             20
Cod. Strang       5'  CT  AGA  ATG  ACG  TAT  ACT  GAC  TGC
nicht cod. Strang 3'       T  TAC  TGC  ATA  TGA  CTG  ACG
```

```
 6    7    8    9   10   11   12   13   14   15
Thr  Glu  Ser  Gly  Gln  Asn  Leu  Cys  Leu  Cys
          30             40             50
ACT  GAA  TCT  GGT  CAG  AAC  CTG  TGC  CTG  TGC
TGA  CTT  AGA  CCA  GTC  TTG  GAC  ACG  GAC  ACG
```

```
16   17   18   19   20   21   22   23   24   25
Glu  Gly  Ser  Asn  Val  Cys  Gly  Gln  Gly  Asn
          60             70             80
GAA  GGA  TCT  AAC  GTT  TGC  GGC  CAG  GGT  AAC
CTT  CCT  AGA  TTG  CAA  ACG  CCG  GTC  CCA  TTG
```

```
26   27   28   29   30   31   32   33   34   35
Lys  Cys  Ile  Leu  Gly  Ser  Asp  Gly  Glu  Lys
          90             100            110
AAA  TGC  ATC  CTT  GGA  TCC  GAC  GGT  GAA  AAG
TTT  ACG  TAG  GAA  CCT  AGG  CTG  CCA  CTT  TTC
```

```
36   37   38   39   40   41   42   43   44   45
Asn  Gln  Cys  Val  Thr  Gly  Glu  Gly  Thr  Pro
          120            130            140
AAC  CAG  TGC  GTT  ACT  GGC  GAA  GGT  ACC  CCG
TTG  GTC  ACG  CAA  TGA  CCG  CTT  CCA  TGG  GGC
```

```
46   47   48   49   50   51   52   53   54   55
Lys  Pro  Gln  Ser  His  Asn  Asp  Gly  Asp ·Phe
          150            160            170
AAA  CCG  CAG  TCT  CAT  AAC  GAC  GGC  GAC  TTC
TTT  GGC  GTC  AGA  GTA  TTG  CTG  CCG  CTG  AAG
```

```
56   57   58   59   60   61   62   63   64
Glu  Glu  Ile  Pro  Glu  Glu  Tyr  Leu  Gln  Stp
          180            190            200
GAA  GAG  ATC  CCT  GAG  GAA  TAC  CTT  CAG  TAA
CTT  CTC  TAG  GGA  CTC  CTT  ATG  GAA  GTC  ATT
```

```
Stp
          210
TAG  AGC  TCG              3'
ATC  TCG  AGC  AGC    T    5'
```

DNA-Sequenz IA:

```
              O
             Met   (Aminosäuren 1-64)
     1    5                              205    210
5' CT AGA ATG  (Nucleotide 9-200)  TAA TAG AGC T      3'
3'       T TAC (complement. Nucl.)  ATT TAC           5'
   XbaI

                                             SstI
```

DNA-Sequenz IB:

```
              O
             Arg   (Aminosäuren 1-64)
     1    5                              205    210
5' CT AGA CGT  (Nucleotide 9-200)  TAA TAG AGC T      3'
3'       T TAC (complement. Nucl.)  ATT ATC           5'
   XbaI                                 SstI
```

DNA-Sequenz IC:

```
              O
             Met   (Aminosäuren 1-64)
                                         205    210
5' AA TTC ATG  (Nucleotide 9-200)  TAA TAG AGC TCG          3'
         G TAC (complement. Nucl.)  ATT ATC TCG AGC AGC T   5'
   EcoRI                                 SalI
```

## DNA-Sequenz II A (HIR-I)

```
                                                    I a

Nucleotid-Nr.                                       10                      20
Cod. Strang          5'   CT   AGA   ATG   ACG   TAT   ACT   GAC   TGC
nicht cod. Strang    3'         T    TAC   TGC   ATA   TGA   CTG   ACG
                          XbaI

                                                          I b
                                                    I c
            30                    40                      50
ACT   GAA   TCT   GGT   CAG   AAC   CTG   TGC   CTG   TGC
TGA   CTT   AGA   CCA   GTC   TTG   GAC   ACG   GAC   ACG


      I b                                           I d
        I c                             I e
            60                    70                      80
GAA   GGA   TCT   AAC   GTT   TGC   GGC   CAG   GGT   AAC
CTT   CCT   AGA   TTG   CAA   ACG   CCG   GTC   CCA   TTG


        I d                                          If
      I e
            90
AAA   TGC   ATC   CTT   G     Bam HI   3'
TTT   ACG   TAG   GAA   CCT   AG       5'
        I f
```

## DNA-Sequenz II B (HIR-II)

### II a

| Nucleotid-Nr. | | | | 100 | | | 110 | | |
|---|---|---|---|---|---|---|---|---|---|
| cod. Strang | 5' | | GA | TCC | GAC | GGT | GAA | AAG | |
| nicht cod. Strang | 3' | BamHI | G | CTG | CCA | CTT | TTC | | |

|  |  | II b |
|---|---|---|

| II a | | | | II c | | | | | |
|---|---|---|---|---|---|---|---|---|---|
| 120 | | | | 130 | | | 140 | | |
| AAC | CAG | TGC | GTT | ACT | GGC | GAA | GGT | ACC | CCG |
| TTG | GTC | ACG | CAA | TGA | CCG | CTT | CCA | TGG | GGC |

|  | II b | | | | | | II d |
|---|---|---|---|---|---|---|---|

| II c | | | | I e | | | | | |
|---|---|---|---|---|---|---|---|---|---|
| 150 | | | | 160 | | | 170 | | |
| AAA | CCG | CAG | TCT | CAT | AAC | GAC | GGC | GAC | TTC |
| TTT | GGC | GTC | AGA | GTA | TTG | CTG | CCG | CTG | AAG |

|  | II d | | | | | | II f |
|---|---|---|---|---|---|---|---|
|  | II e | | | | | II g | |

| 180 | | | | 190 | | | 200 | | |
|---|---|---|---|---|---|---|---|---|---|
| GAA | GAG | ATC | CCT | GAG | GAA | TAC | CTT | CAG | TAA |
| CTT | CTC | TAG | GGA | CTC | CTT | ATG | GAA | GTC | ATT |

|  | II f | | | | | | IIh |
|---|---|---|---|---|---|---|---|

II g

| 210 | | | | | |
|---|---|---|---|---|---|
| TAG | AGC | TCG | Sal I | 3' | |
| ATC | TCG | AGC | AGC | T | 5' |

II h

**Patentansprüche**

**Patentansprüche für folgende Vertragsstaaten BE, CH, DE, FR, GB, IT, LI, LU, NL, SE**

1. Verfahren zur Herstellung eines Polypeptids mit Hirudinaktivität der allgemeinen Formel I

$$(X)_{m-1}\text{-}X^1\text{-}C\text{-}Tyr\text{-}D\text{-}Asp\text{-}Cys\text{-}E\text{-}Glu\text{-}Ser\text{-}Gly\text{-}Gln\text{-}Asn\text{-}Leu\text{-}Cys\text{-}Leu\text{-}Cys\text{-}Glu\text{-}Gly\text{-}Ser\text{-}$$

$$Asn\text{-}Val\text{-}Cys\text{-}Gly\text{-}Gln\text{-}Gly\text{-}Asn\text{-}Lys\text{-}Cys\text{-}Ile\text{-}Leu\text{-}Gly\text{-}Ser\text{-}Asp\text{-}F\text{-}G\text{-}Lys\text{-}Asn\text{-}Gln\text{-}Cys\text{-}$$

$$Val\text{-}Thr\text{-}Gly\text{-}Glu\text{-}Gly\text{-}Thr\text{-}Pro\text{-}Lys\text{-}Pro\text{-}Gln\text{-}Ser\text{-}His\text{-}Asn\text{-}Asp\text{-}Gly\text{-}Asp\text{-}Phe\text{-}Glu\text{-}H\text{-}Ile\text{-}$$

$$Pro\text{-}Glu\text{-}Glu\text{-}Tyr\text{-}Leu\text{-}Gln\text{-}(Z)_n\text{-}OH$$

(I)

in welcher

| | | |
|---|---|---|
| m | | = 0 - 50, |
| n | | = 0 - 100 und |
| X und $X^1$ | | für gleiche oder verschiedene Reste genetisch codierbarer Aminosäuren stehen, |
| C | | Thr, Val, Ile, Leu oder Phe, |
| D | | Thr, |
| E | | Thr, |
| F | | Gly, |
| G | | Glu, |
| H | | Glu bedeuten, und |
| Z | | für gleiche oder verschiedene Reste genetisch codierbarer Aminosäuren steht, |

dadurch gekennezeichnet, daß man in ein Expressionsplasmid ein Gen einbaut, das für ein Polypetid der Formel I codiert, wobei weitere der Aminosäuren entfallen oder durch (eine) andere genetisch codierbare Aminosäure(n) ersetzt werden kann (können), wobei die Herstellung der folgenden Polypeptide ausgenommen ist, in denen

a) $X^1$ und C beide Val oder

b) $X^1$ Ile und C Thr bedeuten oder

c) N-terminal Aminosäuren bis zu Position 10 eliminiert sind.

2. Verfahren nach Anspruch 1, dadurch gekennzeichnet, daß man ein Gen einbaut, dessen nicht-codierbarer Strang mit dem codierenden Strang des für das Polypeptid der Formel I codierenden Gens hybridisiert.

3. Verfahren nach Anspruch 1 oder 2, dadurch gekennzeichnet, daß das Gen chemisch, vorzugsweise nach dem Phosphitverfahren, synthetisiert wird.

4. Verfahren nach einem oder mehreren der vorhergehenden Ansprüche, dadurch gekennzeichnet, daß das Gen im Anschluß an das Codon für die carboxyterminale Aminosäure zwei Stop-Codons enthält.

5. Verfahren nach einem oder mehreren der vorhergehenden Ansprüche, dadurch gekennzeichnet, daß das Gen der DNA-Sequenz I in Tabelle 1 entspricht.

6. DNA-Sequenzen I, IA, IB, IC, IIA und IIB entsprechend den Tabellen 1 bis 4.

7. Fusionspeptid, dadurch gekennzeichnet, daß es ein Polypeptid gemäß Anspruch 1 enthält und vorzugsweise sein bakterieller Anteil die Aminosäuresequenz der β-Galactosidase ganz oder teilweise enthält.

8. Hybridplasmid, dadurch gekennzeichnet, daß es eine DNA-Sequenz enthält, die für das Polypeptid der Formel I codiert.

9. Wirtsorganismus, vorzugsweise E. coli, der ein Hybridplasmid nach Anspruch 8 enthält.

**Patentansprüche für folgenden Vertragsstaat : AT**

1. Verfahren zur Herstellung eines Polypeptids mit Hirudinaktivität der allgemeinen Formel I

$$(X)_{m-1}\text{-}X^1\text{-}C\text{-}Tyr\text{-}D\text{-}Asp\text{-}Cys\text{-}E\text{-}Glu\text{-}Ser\text{-}Gly\text{-}Gln\text{-}Asn\text{-}Leu\text{-}Cys\text{-}Leu\text{-}Cys\text{-}Glu\text{-}Gly\text{-}Ser\text{-}$$

$$Asn\text{-}Val\text{-}Cys\text{-}Gly\text{-}Gln\text{-}Gly\text{-}Asn\text{-}Lys\text{-}Cys\text{-}Ile\text{-}Leu\text{-}Gly\text{-}Ser\text{-}Asp\text{-}F\text{-}G\text{-}Lys\text{-}Asn\text{-}Gln\text{-}Cys\text{-}$$

$$Val\text{-}Thr\text{-}Gly\text{-}Glu\text{-}Gly\text{-}Thr\text{-}Pro\text{-}Lys\text{-}Pro\text{-}Gln\text{-}Ser\text{-}His\text{-}Asn\text{-}Asp\text{-}Gly\text{-}Asp\text{-}Phe\text{-}Glu\text{-}H\text{-}Ile\text{-}$$

$$Pro\text{-}Glu\text{-}Glu\text{-}Tyr\text{-}Leu\text{-}Gln\text{-}(Z)_n\text{-}OH$$

(I)

in welcher

| | |
|---|---|
| m | = 0 - 50, |
| n | = 0 - 100 und |
| X und $X^1$ | für gleiche oder verschiedene Reste genetisch codierbarer Aminosäuren stehen, |
| C | Thr, Val, Ile, Leu oder Phe, |
| D | Thr, |
| E | Thr, |
| F | Gly, |
| G | Glu, |
| H | Glu bedeuten, und |
| Z | für gleiche oder verschiedene Reste genetisch codierbarer Aminosäuren steht, |

dadurch gekennezeichnet, daß man in ein Expressionsplasmid ein Gen einbaut, das für ein Polypetid der Formel I codiert, wobei weitere der Aminosäuren entfallen oder durch (eine) andere genetisch codierbare Aminosäure(n) ersetzt werden kann (können), wobei die Herstellung der folgenden Polypeptide ausgenommen ist, in denen

a) $X^1$ und C beide Val oder

b) $X^1$ Ile und C Thr bedeuten oder

c) N-terminal Aminosäuren bis zu Position 10 eliminiert sind.

2. Verfahren nach Anspruch 1, dadurch gekennzeichnet, daß man ein Gen einbaut, dessen nicht-codierbarer Strang mit dem codierenden Strang des für das Polypeptid der Formel I codierenden Gens hybridisiert.

3. Verfahren nach Anspruch 1 oder 2, dadurch gekennzeichnet, daß das Gen chemisch, vorzugsweise nach dem Phosphitverfahren, synthetisiert wird.

4. Verfahren nach einem oder mehreren der vorhergehenden Ansprüche, dadurch gekennzeichnet, daß das Gen im Anschluß an das Codon für die carboxyterminale Aminosäure zwei Stop-Codons enthält.

5. Verfahren nach einem oder mehreren der vorhergehenden Ansprüche, dadurch gekennzeichnet, daß das Gen der DNA-Sequenz I in Tabelle 1 entspricht.

**Claims**

16

**Claims for the following Contracting States : BE, CH, DE, FR, GB, IT, LI, LU, NL, SE**

1. A process for the preparation of a polypeptide having hirudin activity, of the formula I

$$(X)_{m-1}\text{-}X'\text{-}C\text{-}Tyr\text{-}D\text{-}Asp\text{-}Cys\text{-}E\text{-}Glu\text{-}Ser\text{-}Gly\text{-}Gln\text{-}Asn\text{-}Leu\text{-}Cys\text{-}Leu\text{-}Cys\text{-}Glu\text{-}Gly\text{-}Ser\text{-}$$
(positions 5, 10, 15)
$$Asn\text{-}Val\text{-}Cys\text{-}Gly\text{-}Gln\text{-}Gly\text{-}Asn\text{-}Lys\text{-}Cys\text{-}Ile\text{-}Leu\text{-}Gly\text{-}Ser\text{-}Asp\text{-}F\text{-}G\text{-}Lys\text{-}Asn\text{-}Gln\text{-}Cys\text{-}$$
(positions 20, 25, 30, 35)
$$Val\text{-}Thr\text{-}Gly\text{-}Glu\text{-}Gly\text{-}Thr\text{-}Pro\text{-}Lys\text{-}Pro\text{-}Gln\text{-}Ser\text{-}His\text{-}Asn\text{-}Asp\text{-}Gly\text{-}Asp\text{-}Phe\text{-}Glu\text{-}H\text{-}Ile\text{-}$$
(positions 40, 45, 50, 55)
$$Pro\text{-}Glu\text{-}Glu\text{-}Tyr\text{-}Leu\text{-}Gln\text{-}(Z)_n\text{-}OH$$
(position 60)

$$(I)$$

in which

| | |
|---|---|
| $m =$ | 0 - 50, |
| $n =$ | 0 - 100, and |
| $X$ and $X^1$ | represent identical or different residues of genetically codable amino acids, |
| C | denotes Thr, Val, Ile, Leu, or Phe, |
| D | denotes Thr |
| E | denotes Thr |
| F | denotes Gly |
| G | denotes Glu |
| H | denotes Glu, and |
| Z | represents identical or different residues of genetically codable amino acids, |

which comprises incorporation into an expression plasmid of a gene which codes for a polypeptide of the formula I, it being possible for other amino acids to be omitted or replaced by (an)other genetically codable amino acid(s), excepting the preparation of the following polypeptides in which

a) $X^1$ and C are both Val or

b) $X^1$ denotes Ile and C denotes Thr or

c) N-terminal amino acids are eliminated up to position 10.

2. The process as claimed in claim 1, wherein the non-coding strand of the incorporated gene hybridizes with the coding strand of the gene coding for the polypeptide of the formula I.

3. The process as claimed in claim 1 or 2, wherein the gene has been synthesized chemically, preferably by the phosphite process

4. The process as claimed in one or more of the preceding claims, wherein the gene contains two stop codons down-stream of the codon for the carboxy-terminal amino acid.

5. The process as claimed in one or more of the preceding claims, wherein the gene corresponds to the DNA sequence I in Table 1.

6. DNA sequences I, IA, IB, IC, IIA and IIB corresponding to Tables 1 to 4.

7. A fusion peptide which contains a polypeptide as claimed in claim 1, and wherein its bacterial fraction preferably contains all or part of the amino acid sequence of $\beta$-galactosidase.

8. A hybrid plasmid which contains a DNA sequence which codes for the polypeptide of the formula I.

9. A host organism, preferably E. coli, which contains a hybrid plasmid as claimed in claim 8.

**Claims for the following Contracting State : AT**

1. A process for the preparation of a polypeptide having hirudin activity, of the formula I

$(X)_{m-1}$-$X^1$-C-Tyr-D-Asp-Cys-E-Glu-Ser-Gly-Gln-Asn-Leu-Cys-Leu-Cys-Glu-Gly-Ser-
Asn-Val-Cys-Gly-Gln-Gly-Asn-Lys-Cys-Ile-Leu-Gly-Ser-Asp-F-G-Lys-Asn-Gln-Cys-
Val-Thr-Gly-Glu-Gly-Thr-Pro-Lys-Pro-Gln-Ser-His-Asn-Asp-Gly-Asp-Phe-Glu-H-Ile-
Pro-Glu-Glu-Tyr-Leu-Gln-$(Z)_n$-OH

(I)

in which

| | |
|---|---|
| m = | 0 - 50, |
| n = | 0 - 100, and |
| X and $X^1$ | represent identical or different residues of genetically codable amino acids, |
| C | denotes Thr, Val, Ile, Leu, or Phe, |
| D | denotes Thr |
| E | denotes Thr |
| F | denotes Gly |
| G | denotes Glu |
| H | denotes Glu, and |
| Z | represents identical or different residues of genetically codable amino acids, |

which comprises incorporation into an expression plasmid of a gene which codes for a polypeptide of the formula I, it being possible for other amino acids to be omitted or replaced by (an)other genetically codable amino acid(s), excepting the preparation of the following polypeptides in which

a) $X^1$ and C are both Val or

b) $X^1$ denotes Ile and C denotes Thr or

c) N-terminal amino acids are eliminated up to position 10.

2. The process as claimed in claim 1, wherein the non-coding strand of the incorporated gene hybridizes with the coding strand of the gene coding for the polypeptide of the formula I.

3. The process as claimed in claim 1 or 2, wherein the gene has been synthesized chemically, preferably by the phosphite process

4. The process as claimed in one or more of the preceding claims, wherein the gene contains two stop codons down-stream of the codon for the carboxy-terminal amino acid.

5. The process as claimed in one or more of the preceding claims, wherein the gene corresponds to the DNA sequence I in Table 1.

**Revendications**
**Revendications pour les Etats contractants suivants : BE, CH, DE, FR, GB, IT, LI, LU, NL, SE**

1. Procédé pour la production d'un polypeptide à activité d'hirudine, de formule générale I

$(X)_{m-1}$-$X^1$-C-Tyr-D-Asp-Cys-E-Glu-Ser-Gly-Gln-Asn-Leu-Cys-Leu-Cys-Glu-Gly-Ser- (positions 5, 10, 15)

Asn-Val-Cys-Gly-Gln-Gly-Asn-Lys-Cys-Ile-Leu-Gly-Ser-Asp-F-G-Lys-Asn-Gln-Cys- (positions 20, 25, 30, 35)

Val-Thr-Gly-Glu-Gly-Thr-Pro-Lys-Pro-Gln-Ser-His-Asn-Asp-Gly-Asp-Phe-Glu-H-Ile- (positions 40, 45, 50, 55)

Pro-Glu-Glu-Tyr-Leu-Gln-$(Z)_n$-OH (position 60)

(I)

dans laquelle

m = 0-50,

n = 0-100 et

X et $X^1$ représentent des restes identiques ou différents d'aminoacides génétiquement codables,

C représente Thr, Val, Ile, Leu ou Phe,

D représente Thr,

E représente Thr,

F représente Gly,

G représente Glu,

H représente Glu, et

Z représente des restes identiques ou différents d'aminoacides génétiquement codables, caractérisé en ce que l'on incorpore dans un plasmide d'expression un gène qui code pour un polypeptide de formule I, les autres aminoacides étant absent ou pouvant être remplacé par un autre (d'autres) aminoacide(s) génétiquement codable(s), à l'exclusion de la production des polypeptides dans lesquels

a) $X^1$ et C représentent l'un et l'autre Val ou

b) $X^1$ représente Ile et C représente Thr ou

c) les aminoacides à l'extrémité N-terminale sont éliminés jusqu'à la position 10.

2. Procédé selon la revendication 1, caractérisé en ce que l'on incorpore un gène dont le brin non codant est hybridé avec le brin codant du gène codant pour le polypeptide de formule I.

3. Procédé selon la revendication 1 ou 2, caractérisé en ce que le gène est synthétisé chimiquement, de préférence selon la méthode au phosphite.

4. Procédé selon une ou plusieurs des revendications précédentes, caractérisé en ce que le gène contient deux codons d'arrêt à la suite du codon codant pour l'aminoacide à l'extrémité carboxy.

5. Procédé selon une ou plusieurs des revendications précédentes, caractérisé en ce que le gène correspond à la séquence d'ADN I dans le tableau 1.

6. Séquences d'ADN I, IA, IB, IC, IIA et IIB correspondant aux tableaux 1 à 4.

7. Peptide fusionné, caractérisé en ce qu'il contient un polypeptide selon la revendication 1, et de préférence son fragment bactérien contient en partie ou en totalité la séquence d'aminoacides de la β-galactosidase.

8. Plasmide hybride, caractérisé en ce qu'il contient une séquence d'ADN qui code pour le polypeptide de formule I.

9. Organisme hôte, de préférence E. coli, qui contient un plasmide hybride selon la revendication 8.

**Revendications pour l'Etat contractant suivant : AT**

1. Procédé pour la production d'un polypeptide à activité d'hirudine, de formule générale I

$$(X)_{m-1}\text{-}X^1\text{-}C\text{-}Tyr\text{-}D\text{-}Asp\text{-}Cys\text{-}E\text{-}Glu\text{-}Ser\text{-}Gly\text{-}Gln\text{-}Asn\text{-}Leu\text{-}Cys\text{-}Leu\text{-}Cys\text{-}Glu\text{-}Gly\text{-}Ser\text{-}$$
$$\overset{5}{\phantom{x}}\qquad\overset{10}{\phantom{x}}\qquad\overset{15}{\phantom{x}}$$

$$Asn\text{-}Val\text{-}Cys\text{-}Gly\text{-}Gln\text{-}Gly\text{-}Asn\text{-}Lys\text{-}Cys\text{-}Ile\text{-}Leu\text{-}Gly\text{-}Ser\text{-}Asp\text{-}F\text{-}G\text{-}Lys\text{-}Asn\text{-}Gln\text{-}Cys\text{-}$$
$$\overset{20}{\phantom{x}}\qquad\overset{25}{\phantom{x}}\qquad\overset{30}{\phantom{x}}\qquad\overset{35}{\phantom{x}}$$

$$Val\text{-}Thr\text{-}Gly\text{-}Glu\text{-}Gly\text{-}Thr\text{-}Pro\text{-}Lys\text{-}Pro\text{-}Gln\text{-}Ser\text{-}His\text{-}Asn\text{-}Asp\text{-}Gly\text{-}Asp\text{-}Phe\text{-}Glu\text{-}H\text{-}Ile\text{-}$$
$$\overset{40}{\phantom{x}}\qquad\overset{45}{\phantom{x}}\qquad\overset{50}{\phantom{x}}\qquad\overset{55}{\phantom{x}}$$

$$Pro\text{-}Glu\text{-}Glu\text{-}Tyr\text{-}Leu\text{-}Gln\text{-}(Z)_n\text{-}OH$$
$$\overset{60}{\phantom{x}}$$

**(I)**

dans laquelle

m = 0-50,

n = 0-100 et

X et $X^1$ représentent des restes identiques ou différents d'aminoacides génétiquement codables,

C représente Thr, Val, Ile, Leu ou Phe,

D représente Thr,

E représente Thr,

F représente Gly,

G représente Glu,

H représente Glu, et

Z représente des restes identiques ou différents d'aminoacides génétiquement codables, caractérisé en ce que l'on incorpore dans un plasmide d'expression un gène qui code pour un polypeptide de formule I, les autres aminoacides étant absent ou pouvant être remplacé par un autre (d'autres) aminoacide(s) génétiquement codable(s), à l'exclusion de la production des polypeptides dans lesquels

a) $X^1$ et C représentent l'un et l'autre Val ou

b) $X^1$ représente Ile et C représente Thr ou

c) les aminoacides à l'extrémité N-terminale sont éliminés jusqu'à la position 10.

2. Procédé selon la revendication 1, caractérisé en ce que l'on incorpore un gène dont le brin non codant est hybridé avec le brin codant du gène codant pour le polypeptide de formule I.

3. Procédé selon la revendication 1 ou 2, caractérisé en ce que le gène est synthétisé chimiquement, de préférence selon la méthode au phosphite.

4. Procédé selon une ou plusieurs des revendications précédentes, caractérisé en ce que le gène contient deux codons d'arrêt à la suite du codon codant pour l'aminoacide à l'extrémité carboxy.

5. Procédé selon une ou plusieurs des revendications précédentes, caractérisé en ce que le gène correspond à la séquence d'ADN I dans le tableau 1.

FIG.1

FIG.2

FIG.3

FIG.4

FIG.5

FIG.6